(19) Europäisches Patentamt
European Patent Office
Office européen des brevets

(11) **EP 4 098 284 A2**

(12) **EUROPEAN PATENT APPLICATION**

(43) Date of publication:
**07.12.2022 Bulletin 2022/49**

(21) Application number: **22177259.3**

(22) Date of filing: **03.06.2022**

(51) International Patent Classification (IPC):
*A61L 9/20* (2006.01)     *B01D 39/08* (2006.01)
*B01D 46/00* (2022.01)     *B01D 53/00* (2006.01)
*B01D 53/86* (2006.01)     *F24F 8/10* (2021.01)
*F24F 8/22* (2021.01)     *F25D 17/04* (2006.01)

(52) Cooperative Patent Classification (CPC):
**A61L 9/205; B01D 39/083; B01D 39/086;
B01D 46/0028; B01D 53/007; B01D 53/8687;
F24F 8/10; F24F 8/22; F25D 17/042;** A61L 2209/14;
A61L 2209/16; B01D 2239/0492

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **03.06.2021 IT 202100014477**

(71) Applicant: **Regia Special Homes S.r.l.
17027 Pietra Ligure (SV) (IT)**

(72) Inventors:
• **RAINATO, RENATA**
  **Pietra Ligure (SV) (IT)**
• **SALVI, GIANLUIGI**
  **Pietra Ligure (SV) (IT)**
• **GANDINI, ALFREDO**
  **Pietra Ligure (SV) (IT)**

(74) Representative: **Karaghiosoff, Giorgio
Alessandro
c/o Praxi Intellectual Property S.p.A. - Savona
Via F. Baracca 1R, 4° piano
"Il Gabbiano"
17100 Savona (IT)**

(54) **AIR FILTERING APPARATUS, METHOD AND SYSTEM**

(57)     The object of the invention is an apparatus for the reduction and/or the removal of chemical/microbiological pollutants in indoor air flowing through said apparatus, which comprises: a first filter medium (4), placed to filter the incoming air (10), an element (5) decelerating the airflow coming from the said first filter medium (4), said decelerating element made in a way to slow down the air flow (10) passing through it, a reaction chamber (T) of a predetermined volume, placed between said first filter medium (4) and said decelerating element (6), said reaction chamber where the air flows to be irradiated with specific radiation (10) and at least a source of radiation inducing photochemical action (8), disposed in such a way to irradiate the walls and the inside of the reaction chamber, preferably with ultraviolet light radiation.

    Furthermore, object of the invention is a system comprising one or a plurality of said apparatuses and a method to implement them, in order to achieve the sanitization of indoor air.

Fig. 1

**Description**

The present invention relates to an air purification apparatus for indoor environments needing sanitization from airborne polluting agents. The invention also relates to systems using one or more of these apparatuses and to the methods to achieve air sanitization abating viruses, bacteria and VOC, useful in other fields of application not directly connected to human health, yet suitable to bring benefits such as slowing down the deterioration of fruit, vegetables and flowers, limiting airborne infections in animal farming, improving control in post-harvest horticultural products and control of health risk of animal husbandry.

The pandemic that has recently hit mankind has highlighted the importance to upgrade the current heat and ventilation air conditioning systems with devices that can deliver air that is not just cleaner but also sanitized.

Vaccines are, together with the guidance and preventive measures adopted by different health organizations throughout the world, the prevailing solution to fight viruses like Covid19, regardless of their capacity to adapt and mutate.

Scope of the present invention is to contribute to the mitigation of airborne pathogen diffusion, required especially indoors in crowded settings with turnover of people, where the air is stagnant or recirculated, possibly conditioned with a highly unbalanced ratio of outside/inside air, as it is in HVACs of civil, commercial, industrial, medical facilities, and on public and private transportation, where the current filtration systems are inadequate to tackle the problem.

The system and method, as well as the apparatus object of the present invention, have been engineered to sanitize air conditioning systems in any sector from viruses and bacteria, with an efficacy of abatement of 99,9% of airborne pathogens of various origins, thus improving indoor air quality; recent studies and surveys on indoor air have shown that its pollution is one of the major health risks causing premature deaths.

A first scope of the present invention is the addiction of one or more devices to entrap and deactivate contaminants inside Air Handling Units of civil, commercial, industrial, medical sectors and on board public/private trasportation, in order to abate pathogens of various kinds, particularly viruses and bacteria.

Integrated into existing AHU/HVAC systems, conventionally built only as air-conditioners with filter, the device turns them into air purifiers, eliminating airborne pathogens, retaining and destroying them as a result of the virucidal power of the photocatalytic oxidation triggered by at least one catalyst activated by electromagnetic radiation in the UV bandwidth.

The sanitizing action is boosted by decelerating elements that slow down the airflow in the irradiation area, exposing it for a longer time to light, preferably light-emitting diodes in the wavelength C.

**STATE OF THE ART**

In the air conditioning systems, also called AHU - Air Handling Unit - or HVAC - Heating, Ventilation and Air Conditioning - the air is blown into a room by a fan inside the unit, and flows through one or more high efficiency filters, named EPA (Efficiency Particulate Air filter - B Group) or HEPA (High Efficiency Particulate Air filter - H Group) defined by the European standard EN 1822-1:2019, that establishes the criteria for calculating their level of efficiency with a method to count the number of particles in liquid or solid aerosol test to classify these filters, in a standard way, according to their performance.

These filters are composed of multilayered mats of microfibers (generally in borosilicate) and aluminium separators. The mats are used to block polluting particles of particulate matter in the airflow to be filtered. The main airborne polluting agents are $CO_2$ (carbon dioxide), $SO_x$ (sulfor oxide), $NO_x$ (nitrogen oxide), CO (carbon monoxide), PAHs (polycyclic aromatic hydrocarbons), VOC (Volatile Organic Compounds), Particulate Matter (PM 2.5, PM 10).

HEPA filters belong to the category of so-called "absolute filters", which also includes ULPA filters (Ultra Low Penetration Air) a term used to state that HEPA and ULPA filters have a high filtration efficiency. In particular, HEPA filters have a filtration efficiency between 85% (H10) and 99.995% (H14), while ULPA filters have a filtration efficiency between 99.9995% (U15) and 99.999995% (U17).

Such filters are classified on the basis of the removal efficiency for particles of 0.3 $\mu$m. A monodispersed aerosol continuously generated by condensation of dioctylphthalate (DOP test) is used to evaluate them, with results accepted for penetration with a coefficient of 0.001. However modern and high-tech the air handling systems with fitted HEPA filters mentioned above may seem, they cannot destroy viruses and bacteria, pathogenic microorganisms of various shapes and sizes ranging from 18 to 300 nm. Decreasing the filter mesh size to retain particles and microorganisms below 0.3 $\mu$m would create an almost impenetrable barrier to the airflow, compromising its flow rate and therefore undermining the efficiency of the AHU-HVAC air conditioning systems. Besides, filtration is limited to the arrestance of pollutants, not to their decomposition, leading to rapid clogging and declining performance of the filters. Furthermore, excessive moisture accumulates on the air-conditioning filters and in ducts, creating a perfect breeding ground for mold and fungal spores, which can be blown back through the ducts in indoor areas, if there isn't a frequent maintenance and replacement of filters, which are expensive and not recyclable. Despite the use of absolute filters in hospitals HVACs, infections associated to Aspergillus Flavus and Niger continue to occur. HEPA don't trap viruses smaller than 0.3 $\mu$m,

that could be released back into the ambient air; they don't stop gases, fumes, odours and other VOC. Some bacteria captured by filters can retain their viability and colonize them; endotoxins from the trapped dead bacterial particles released from free DNA can potentially be blown out with the air flow, causing allergies and irritations of the respiratory system.

Another technology used for the removal of noxious particles in the air is Cold Plasma Generator of Negative Air Ions. Its adverse effects - by-products such as ozone, carboxylic acid and free radicals - have been investigated by the American Society of Heating, Refrigerating and Air-conditioning Engineers ASHRAE, that confirmed they may cause deleterious health effects in developing lungs of children and trigger asthmatic reactions. A study of the Environmental Health Sciences of Peking University using ionizers in classes among children found that they decreased PM concentration in the air, but these benefits were overcast by a deterioration of the heart rate variability HRV. There is therefore a huge need for an air sanitizing device that can be integrated into pre-existing or new HVAC systems or purposely built as a stand-alone unit in order to decontaminate indoor air from noxious microorganisms smaller than 0.3 $\mu$m, improving the quality of air without restricting the airflow and/or stopping air recirculation.

Patent application IT201800000622A1 of Nextmaterials S.R.L. published on 08/07/2019 describes a filtering material that, instead of tightening the microfiber mesh in a bidimensional pattern, it's weaved three-dimensionally to extend the air flow path inside the medium of a corrugated cardboard combined with photocatalysts activated by UV, obtaining NOx abatement.

Titanium dioxide, owing to its large bandgap around 3.2 eV, corresponding to UV bandwidth, provides a sufficient amount of photons to trigger a photocatalytic reaction. The synergy TIO2-Light generates Reactive Oxygen Species (ROS) including the hydroxyl radical •OH, the main reactant in the photocatalytic process triggering the virucidal and bactericidal activity.

These highly reactive radicals that form on the surface of the titanium coated material under the effect of ultraviolet light prevent the proliferation of microorganisms and biofouling. Initially, the high oxidizing power of the radicals causes damage to the external cell wall of the microorganisms that come into contact with the photocatalytic surface. With no more protection from the external environment, the internal cytoplasmic membranes are subsequently attacked, causing an outflow of intracellular fluids that leads the cell to rapid death. This powerful biocidal effect has suggested the use of titanium dioxide-based nano-coatings on three-dimensional filters exposed to the irradiation of ultraviolet light in the C band (UV-C), in order to boost the efficacy of air decontamination, increasing exposure time of the airflow to the disinfecting action of the photocatalytic process.

The patent application CN111514670A published on 11/08/2020 with priority of 01/02/2019 anticipates the use of the titanium dioxide photocatalysis on others' filters (with indication of the mesh-size of the ceramic material of the filter, irradiated by UV to obtain virucidal effect). In other applications known to the state of the art use conventional UV germicidal lamps with low-pressure mercury, generating Ozone as a by-product among others, harmful when breathed. Therefore, in a more beneficial use of the technology, UVC LEDs are preferable as source of ultra-violet light needed to carry out the process. It is known that the virucidal function is related both to the exposure time and the surface area coated with the photocatalytic nanomaterial, specifically calculated to achieve the desired percentage of 99% abatement of viruses and bacteria such as MERS, SARS, and current Covid19.

## SUMMARY OF THE INVENTION

Scope of the present invention is:

1) to ensure an efficient and rapid system to abate 99,9% of viruses and bacteria in the first air pass in heating, ventilation and air conditioning systems in public, commercial, industrial, medical facilities and on-board public and private means of transport etc., integrated into air handling units and similar equipment operating with low-medium-high airflow rates;
2) to overcome the inadequacies of conventional filtration techniques, that are limited to the arrestance of airborne viruses and bacteria, by means of devices enabling the exposure of those microorganisms to the virucidal action of UV coupled with the photocatalytic process in order to oxidize them;
3) to ensure that air purification is achieved without any adverse effects due to generation of noxious by-products.

To implement these air sanitizing apparatuses it is necessary to determine their right capacity relative to the room size, the number of people, the type of ventilation/conditioning system where they have to be fitted, its ductwork and - if any-its number of air inlet and outlet vents. It is necessary to estimate the required airflow rate and, once calculated the duct cross-section, the speed of the airflow to be processed.

The air flow, moved by a blower, can be measured assessing Density (d), fluid Velocity (V), cross-sectional area (S), humidity and the presence of other gases.

Density should be calculated according to Pressure (P) and Temperature (T), but where ventilation and air extraction are concerned, an approximation of 1,199Kg/m3 of Density measured at 20°, 50% RH Relative Humidity and Pressure 1.013 hPa is regarded to as sufficient.

Volume flow Q is often measured in m3/h with the formula:

$$Q [m3/h] = V [m/s] x S [m2] x 3.600.$$

Assuming constant density and taken as known the presence of other gases or vapors, the flow rate depends only on the air velocity. One of the systems to detect it is based on the measurement of the dynamic pressure (Pd) by means of Manometers.

The velocity is then calculated with the formula:

$$V [m/s] = K\sqrt{(PD [Pa])}$$

where K is a dimensionless coefficient experimentally obtained.

In compliance with the UNI standard EN 12599 on testing, in the specific case of this invention, to assess the airflow velocity in the system, Pitot tubes can be used, with procedures defined by the air duct cross-section with the formula:

$$V[m/s] = S \text{ pitot factor}] \sqrt{(2x \Delta P [\text{dynamic pressure in Pa}] / rho [\text{air density in kg/}])}$$

By way of example of velocity obtained in the conditions provided by the standard, we can calculate that through a section of 0.5 m2 with a flow rate of e.g. equal to 2,500 /h of a AHU/HVAC, the air flow speed is approximately 1.39 m / s.

To achieve the scope of the present invention these parameters must be taken into account:

- Pa [air flow rate m3 /h];

- Rho [air density in Kg / m3 based on operating condition values such as Temperature, Humidity and Atmospheric Pressure];

- S [air duct surface in m2];

- Pp [Pressure drop m3 / h detected downstream of each apparatus];

- Va [air flow Velocity in m/s];

- Tf [Time needed for bactericidal and virucidal action];

- Pl [Power of UV-C LEDs in mJ or mW • sec];

- Duv [UV-C dose required for bactericidal and virucidal action];

- Df [Filter size in Cm2];

- Dc [Duct size in Cm2].

These data will allow to implement the dimensioning method of the apparatuses object of the present invention.

This method is based on determining the size of the filters for these to be compatible with the inlet and outlet ducts, implementing a shape that allows the filtering surface to be greater than the surface of the duct in which it is positioned, with the formula:

$$Df [Cm2] = Dc • (Pa / Pp)$$

Such formula is used to compensate the pressure drop Pa of ventilation, heating and conditioning systems.

Regarding the amount of ultraviolet radiation on the filter surface, necessary to inactivate viruses, bacteria and VOC, it is calculated according to the equation J / cm2 = W sec / cm2, the stronger the irradiation the shorter the exposure time needed for inactivation. In the specific case, fixing the estimated time at 1 hour, 2mW/cm2 was calculated at a conical coverage distance > 40 mm <50 mm.

In regard to the estimated time of contagion in an indoor environment in given conditions, a study of the Massachusetts Institute of Technology fixes at 72 minutes the safe time before which the risk of infection becomes unacceptably high; to achieve air purification in a time before this threshold, the right ratio is: led UVC 270/290 nm - 2mw/cm2. Exposure time required for the virucidal action of photocatalysis is given by this formula:

$$Tf[h] = [(Duv \cdot Df)/Pl]/3.600.$$

Compared to known state-of-the-art filtering systems, the invention introduces the advantage of reducing the risk that pathogenic microorganisms - especially bacteria and molds but also viruses, e.g. SARS viruses, MERS or the recent COVID19 - could linger in various parts of the air-conditioning systems, and potentially be re-released into the indoor air as they are entrapped on the filters but not entirely destroyed, whereas, thanks to the specific photocatalytic reaction introduced, they are mostly annihilated.

The applicant found that conventional devices using photocatalytic oxidation have a low efficacy and a low percentage of abatement of microorganisms under real world conditions, as the velocity and dynamics of the air where they float allows a partial reduction only near the UV source and the photocatalytic surface.

The photocatalytic reaction is all the more effective the longer the contact time between the photocatalytic surface and the pathogens exposed to it, and therefore it will be most effective if these are blocked on a surface suitable for irradiation rather than when they circulate freely in the air in the form of gas or aerosol.

Conveniently, increasing time exposure to UV irradiation allows to reduce its intensity and add safety in the use of this apparatus in regard to the health risk induced by UV-C dosages failing to comply with European standard UN 62471.

Conveniently, promoting the use of sanitizing devices, instead of conventional filtering devices, enables a reduction of the air-conditioning units maintenance cost, in addition to chemical surface disinfection costs; the photocatalytic filtering materials used in the invention are easily washable, reusable and recyclable with a minimal environmental impact and great benefit to indoor air quality.

Furthermore, it was discovered how the use of the apparatus, specifically in the embodiment with TI02 as a catalyst activated with UVC, has an unexpected effect with regard to the reduction of ethylene and other compounds released by fruit and vegetables during their ripening, besides abating their microbial load.

Consequently, this specific use suggests a further advantage that allows to monitor and control the ripening and decay of fruit and vegetables in storage facilities, prolonging their shelf-life and/or minimizing the harmful effects of polluted air inside the cold rooms where the commodities are kept.

The use of the devices in intensive farming mitigates the load of airborne bacteria and viruses noxious to the animals that host them and is particularly beneficial to reduce sow and piglets mortality due to pathogens outbreaks.

The use of the device is also important in milk collection chambers to decontaminate them from airborne viruses and bacteria.

## BRIEF DESCRIPTION OF THE DRAWINGS

These and other characteristics of the present invention will become more apparent from the preferred embodiments of the invention, by way of a non-limitative example, described below with reference to the accompanying drawings:

Fig.1: AHU Air Handling Units placed inside public, commercial, industrial, medical facilities and on board public and private transportation;

Fig.2: Air Conditioning Schematic Diagram of a common Heating, Ventilation and Air-conditioning system according to the state-of-the-art;

Fig.3: schematic diagram of the air sanitizing apparatus and system object of the present invention, according to an embodiment comprising an apparatus arranged in a central chamber and four apparatuses substantially equal arranged closed to air outlets;

Fig.4: schematic diagram of the filtering apparatus and its casing to shield UV-C for external protection;

Fig.5: schematic diagram of the filtering apparatus irradiated by UVC LEDs placed on two sides and its casing shielded for external protection;

Fig.6: schematic diagram of the air sanitizing apparatus filter as shown in section A-A

Fig.7: schematic diagram of the filter in an extended embodiment of the filtering surface with alveolar section as shown in section C-C.

## DESCRIPTION OF SOME PREFERRED EMBODIMENTS

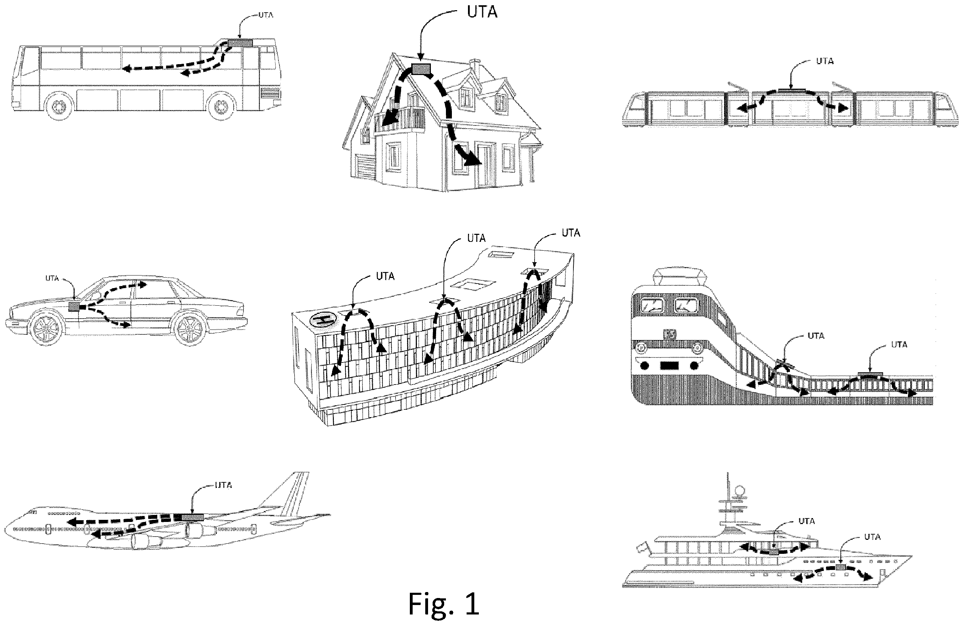

With reference to Fig.1, those shown are only few of many potential installations where the present invention advantages can be exploited to improve indoor air, either integrating or replacing the existent AHU.

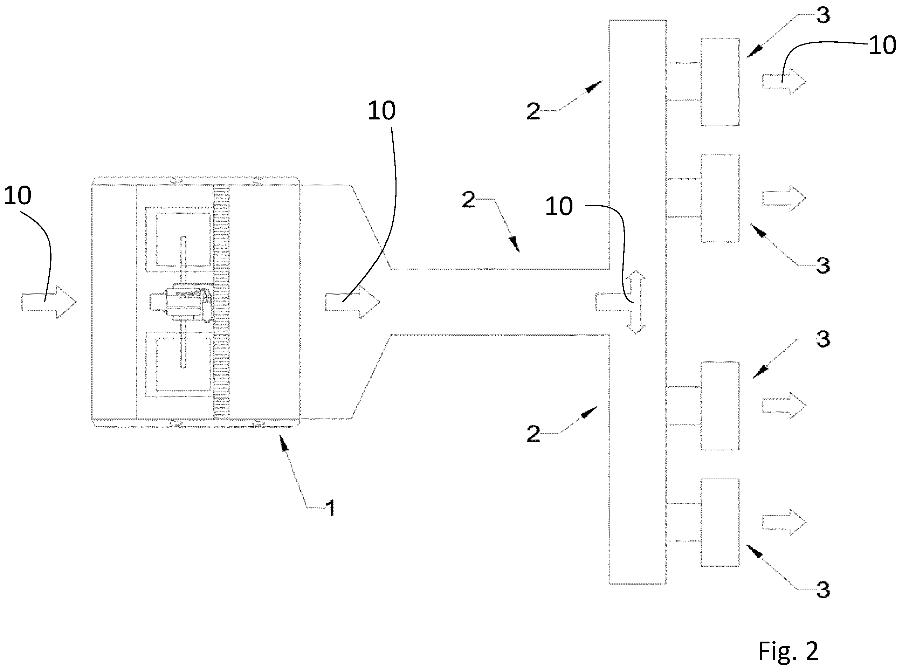

With reference to Fig.2 a typical HVAC system is shown, comprising a plurality of parts, including filters. Such a system comprises an air handler unit 1, ductwork 2 to distribute the air from main area to various rooms, vents 3, openings through which the air is released.

Air intake before the handling takes place in a different area from the air outlet, not a limit for the invention, that is applicable also when there is a percentage of recirculation air, namely air taken from the same room where it will be released. This specific configuration is important in the case of humidity and temperature-controlled environments in the food chain, when ethylene concentrations in the atmosphere need to be reduced to preserve fruit stored in the cold rooms, without air leakage to the outside.

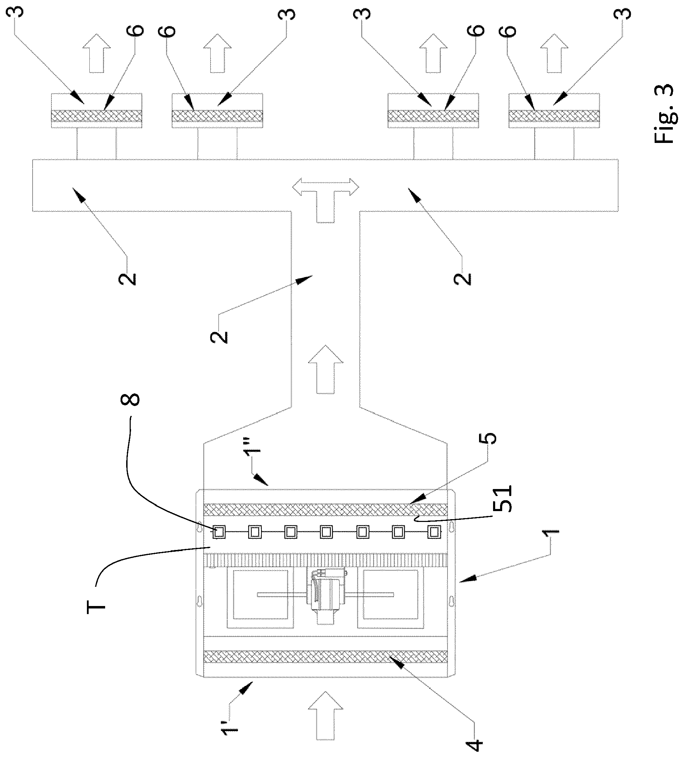

With reference to Fig.3, one possible embodiment of a AHU and an apparatus object of the present invention is shown: at the HVAC/AHU inlet a coarse filter is applied in order to pre-filter the airflow; a decelerating element 5 is applied where the air flows forward, element which is part of a reaction chamber T together with the walls of the casing 9 of unit 1 and filter 4.

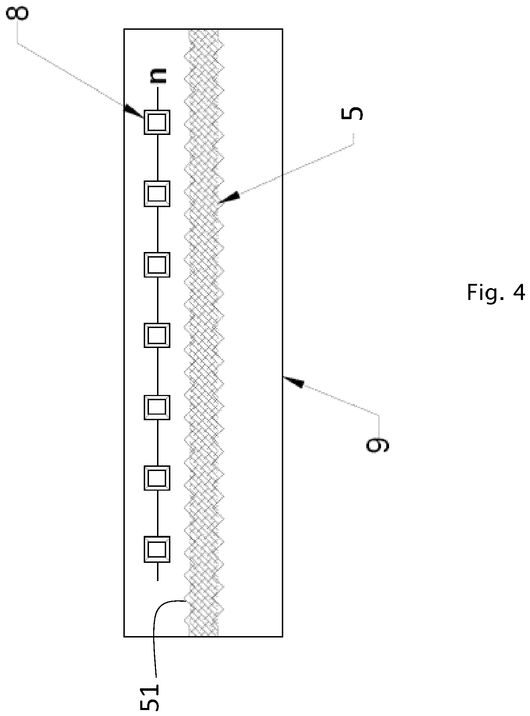

The walls of the casing 9, visible also in fig.4, prevent leakage of air (leading to pressure drop and variation of air flow velocity) and are appropriately shielded to protect human eyes and skin from UVC exposure.

In the reaction chamber T, pressure Pa is increased and airflow is decreased due to the decelerating element. Thus, the advantage achieved is that the longer the air lingers, the longer is its time exposure to UVC irradiation inside the reaction chamber T. In this specific embodiment the radiation source comprises a determined number of UVC LEDs (which could be more than shown in the simplified diagram) whose range and placement is made according to claimed values and methods.

The air decelerating element exposes to the reaction chamber T at least one surface 51 coated with at least one photocatalytic nanomaterial, preferably titanium dioxide, activated by the radiation source in order to decompose the chemical bonds of airborne polluting compounds inside the chamber T.

Using titanium dioxide is advantageous because it is a nanomaterial capable of triggering photocatalytic oxidation, which leads to the abatement of PM, VOC Volatile Organic Compounds and pathogenic microorganisms, bacteria and viruses of various sizes (18 to 300 nm.) and smaller than 0,3 micron.

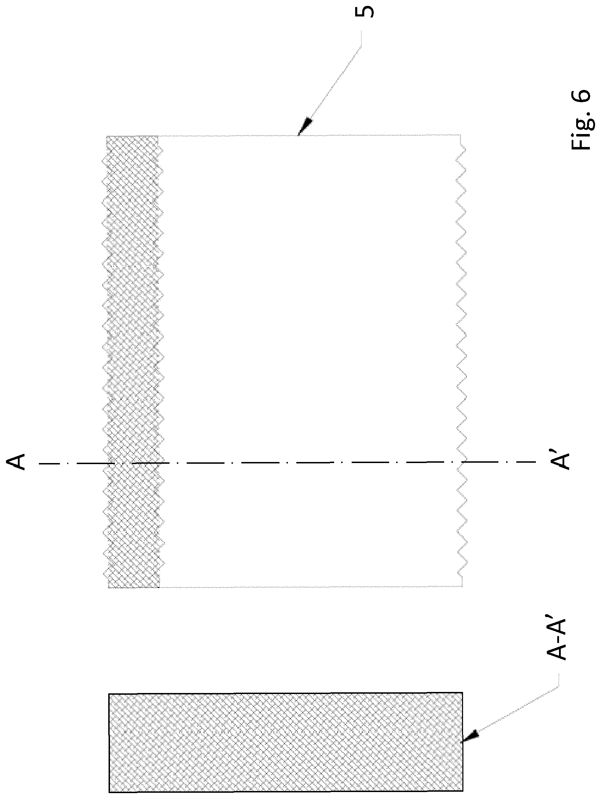

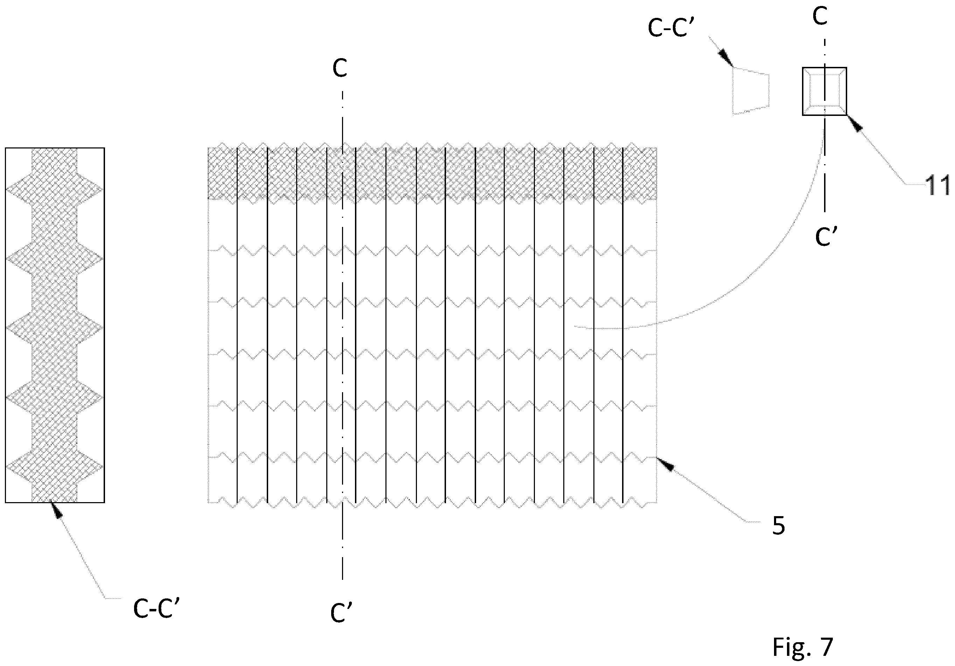

As described in Fig.6 and 7, the invention envisages variants of the decelerating element in the shape of a multiple layers filter, with uniform or alveolar structure made of alveoli 11 with a trapezoidal cross-section.

Regarding the light source 8, preferably UV are used, generated by one or more LEDs configured to emit wavelength light between 100 and 400 nm, preferably in the wavelength range of 200 and 380; the intensity required is between 0,1 mW/cm2 and 10mW/cm2 and preferably 2mW/cm2.

In a different embodiment, not shown here, the device comprises an apparatus used to produce a forced air flow, preferably a centrifugal fan, disposed to draw or blow the air flow through the first filter media, then through the reaction chamber and the decelerating element. The fan can operate in addition to the existing system to compensate the pressure drop that occurs slowing down the air flow speed in the apparatus.

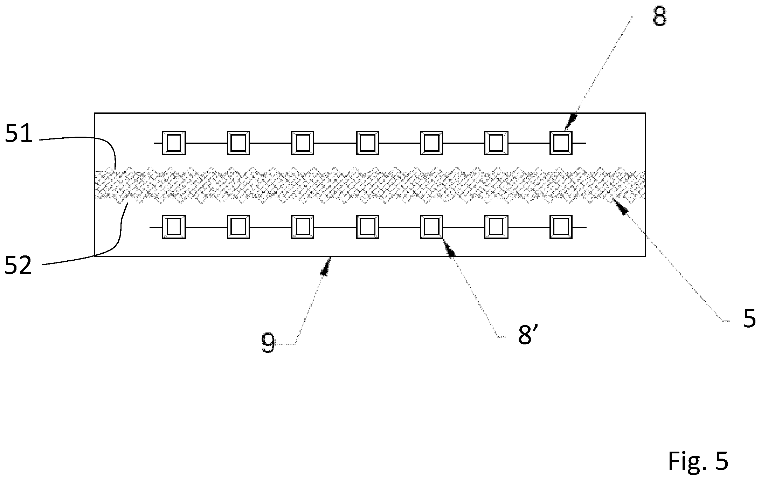

As shown in Fig.5, in another embodiment, freely combinable with other embodiments of the invention, an additional radiation source 8' is fitted, that can be in the same range of light source 8 inside chamber T, disposed to radiate toward the decelerating element where the air flow passes through.

Furthermore, Fig.3 displays the system configured with air distribution ducts, characterized by comprising the installation of additional devices for air filtration and disinfection already described 4,5,8, placed near the outlets 3 where the sanitized air flows into the rooms.

Placement of the devices in the end duct terminal can be alternative or in addition to the placement in the central area, for example in the intermediate section of the ducts 2 or in any derivative zones or any other combinations.

The choice as to where the apparatuses are to be installed inside the system is made according to every single system structure, standard design aspect and potential retrofit of pre-existing systems.

The figures describing the invention are to be considered non-exhaustive and merely examples of its embodiments, as different variants can be potentially implemented by those skilled in the art, without for this reason infringing the field of protection of the same invention, defined by the enclosed claims.

**Claims**

1. Apparatus for the abatement and/or removal of airborne chemical, physical, microbiological polluting particles drifting in an ambient air (10) flowing through said apparatus, said apparatus comprising:

   - A first filtering element(4), arranged so as to be initially crossed by said air flow (10) entering the apparatus;
   - a decelerating element (5) to decelerate the airflow coming from the first filtering element (4), said decelerating element made to decelerate the airflow (10) moving through it;
   - a photocatalytic reaction chamber (T) of pre-determined volume, placed between said first filtering element (4) and said decelerating element(6), said chamber irradiated by a specific radiation and crossed by the airflow (10);
   - at least one source of photochemical radiation arranged to irradiate inside and on the surface walls of said chamber, preferably with UV radiation;
   said decelerating element (5) increasing the lingering time of the ambient air inside the chamber (T) toward which at least one surface (51) of the decelerating element is exposed, being the surface (51) coated with at least one photocatalytic nanomaterial, preferably titanium dioxide, activated by said photochemical radiation source (8) in order to catalyze the oxidation process of the polluting particles.

2. Apparatus according to claim 1, wherein the decelerating element (5) comprises a plurality of superimposed layers along an axis preferably coaxial to the angle of incidence of the air flow, where two or more layers, having an alveolar structure with alveoli (11), are staggered between each other so as to generate branches or divisions of the flow entering a layer into at least two parallel flows in the subsequent layer or layers.

3. Apparatus according to claims 1 or 2, wherein the decelerating element (5) comprises an air filter, composed of a plurality of superimposed filtering layers along an axis which is preferably coaxial to the angle of incidence of the air flow and is optionally provided with at least one internal conveyance and filtration pathway for the airflow to pass through the plurality of said filtering layers.

4. Apparatus according to one or more of the preceding claims **characterized in that** it comprises at least one element for producing a forced current of air arranged and configured so as to force, by suction or by compression, the passage of air (10) in sequence through the first filtering element, through the reaction chamber and through the decelerating element.

5. Apparatus according to one or more of the preceding claims wherein said radiation source (8) is of ultraviolet type and comprises one or more LEDs configured as to emit light having a wavelength included between 100 and 400 nanometers and preferably in the bandwidth between 200 and 380 nanometers, with intensity between 0.1 mW/cm$^2$ and 10mW/cm$^2$ and preferably equal to 2mW/cm$^2$.

6. Apparatus according to one or more of the preceding claims comprising an additional radiation source (8'), preferably of the type indicated in claim 5, disposed as to irradiate a second surface of the decelerating element (52) from which the previously filtered air flows out.

7. System for the purification of indoor air of one or more rooms, comprising:

   - one or more ventilation devices to induce ambient air (10) to circulate or recirculate toward one or more spaces;
   - a duct network (2), for the movement of said ambient air flow, articulated along one or more pathways from at least one central air inlet area to one or more air outlet areas in one or more rooms;
   - a central unit of handling and filtration comprising one or more apparatuses according to one or more of the preceding claims, arranged for the indoor air (10) flow to pass through it to reduce and/or remove its biological and/or chemical polluting particles.

8. System according to claim 7 **characterized in that** it comprises at least one more handling and filtration apparatus (6), according to at least one of the claims from 1 to 6, placed next to the air outlets towards the rooms and/or in any position along the said pathways between the air inlet and the air outlet;

9. Method for the reduction and/or removal of airborne chemical and/or microbiological polluting particles diffused in an ambient airflow channelled inside a ductwork to one or more rooms, comprising the steps of:

   - calculating the volume of those rooms;
   - selecting at least one part of duct of said ductwork;
   - calculating the physical size and section of said duct part;
   - determining the physical characteristics of ambient air, including pressure, temperature, relative humidity, density;
   - determining the ambient air fluid dynamics of the airflow flowing in that part of the duct;
   - reducing and/or removing chemical and/or microbiological pollutants by means of one or more apparatuses, according to one or more of the preceding claims, varying one or more of the following parameters:

      a) irradiation intensity and chamber reaction dimensions
      b) irradiation exposure time (Tf)
      c) pressure drop (Pp)detected downstream each apparatus
      d) UVC dosage to achieve viruses and bacteria decomposition
      e) size of the decelerating element in terms of the surface area exposed to the airflow flowing in the ducts.

10. Use of the apparatus for the reduction and/or removal of chemical and/or microbiological pollutants according to one or more of the preceding claims from 1 to 6 for the purification of controlled atmosphere of food products preservation systems, in particular fruit and vegetables, aimed at the reduction of chemical and/or bacterial agents generated during the ripening process and storage.

11. Ambient atmosphere purification system in cold rooms or storage facilities for the preservation of fruit and vegetables comprising:

   - one or more ventilation devices to move or recirculate ambient air, towards said cold rooms or storage facilities;
   - a duct network for the ambient air to pass along one or more pathways from at least one central air inlet area to one or more air outlet areas;
   - a central unit of air handling and filtration comprising one or more apparatuses according to one or more of the apparatus preceding claims, arranged for the indoor airflow to pass through it in order to reduce and/or remove its content of biological and/or chemical polluting particles.

12. Method for the dosing, reduction and/or removal of chemical and/or microbiological agents present in the controlled atmosphere of cells or storage facilities, by means of an ambient airflow flowing inside a ductwork toward said cells or facilities, comprising the following steps of:

   - calculating the volume of those spaces;
   - selecting at least one part of a duct of said ductwork
   - calculating the physical size and section of said duct part
   - determining the physical characteristics of ambient air, including pressure, temperature, relative humidity, density
   - determining the ambient airflow fluid dynamics inside that part of the duct;
   - determining the type of chemical and/or biological particles to be removed and the type of fruit and vegetable to be preserved;
   - reducing and/or removing chemical and/or microbiological pollutants by means of at least one apparatuses according to one or more preceding claims, having calculated the intensity of irradiation needed and the volume of the reaction chamber.

EP 4 098 284 A2

Fig. 1

Fig. 2

EP 4 098 284 A2

Fig. 3

EP 4 098 284 A2

Fig. 4

Fig. 5

5

A ⎯·⎯·⎯·⎯·⎯·⎯·⎯·⎯·⎯·⎯·⎯·⎯·⎯ A'

A-A'

Fig. 6

EP 4 098 284 A2

C-C'

C

C-C'

C

C'

C'

11

5

Fig. 7

**EP 4 098 284 A2**

**REFERENCES CITED IN THE DESCRIPTION**

**Patent documents cited in the description**

- IT 201800000622 A1
- CN 111514670 A